# EUROPEAN PATENT APPLICATION

(11) **EP 1 752 190 A1**
(43) Date of publication of application: **14.02.2007**
(21) Application number: 06118619.3
(22) Date of filing: 08.08.2006
(51) Int. Cl.: A61M 37/00

(54) **Medical apparatus for cutaneous administration of mendicaments**

(30) Priority: 12.08.2005 BR PI0503545
(71) Applicant: C.I.R.C.E S.R.L., 21052 Busto Arsizio VA (IT)
(72) Inventor: BONIZZONI, Enrico, 21052, BUSTO ARSIZIO (VARESE) (IT)
(74) Representative: Ponzellini, Gianmarco

(57) **Abstract**

A medical apparatus (1) for cutaneous administration of medicaments consisting of an energy emitter (9) active on the molecules of a medicament. The energy emitter (9) can be a laser light generator (11) acting on the polar and non-polar molecules of said medicament. A medicamentous solution (2) disposed between the energy emitter (1) and the skin region to be treated (10) consists of an aqueous matrix and of said medicament. The solution (3) is brought below the solidification temperature and then into abutment with the skin region to be treated (10).

## Description

The present invention relates to a medical apparatus for cutaneous administration of medicaments, of the type comprising the features recited in the preamble of claim 1.

The invention is concerned with the medical sector and in particular (although not in a limiting sense) it relates to physiotherapy treatments carried out be means of laser light sources, better known as "laser therapies". These treatments are particularly used in the field of the aesthetic medicine and in therapeutic actions of the anti-inflammatory, edema-preventing, and analgesic type, for wound healing and for improving circulation disorders.

It is known that the "laser therapy" merely consists in directing a laser light beam emitted from an emitter of radiation of suitable wavelength, towards the skin region to be treated and close thereto.

However, this type of treatment sometimes has drawbacks because it is not always convenient for and proportionate to all pathologies. In fact, the mere action of the laser light is not always efficient enough to treat all the different pathologies that may arise.

Two types of alternative treatments can be presently used in order to face this problem.

The first treatment known as electrophoresis consists in applying an electric current to the skin region to be treated. This electric current is emitted by means of two electrodes disposed on opposite sides of the skin region to be treated.

On the contrary, in the second treatment the well known benefits of the electrophoresis therapy (application of electric currents passing through the region to be treated) are provided to be associated with the current capability of carrying medicamentous substances through the skin.

These treatments are carried out by devices of known type consisting of an electric-energy emitter acting on a medicamentous substance disposed close to the region to be treated. In detail, these devices comprise two electrodes that are disposed on opposite sides of the skin portion of the patient under therapy. A drug generally consisting of a medicament solution is interposed between one of the electrodes and the skin region. The action of the electric current passing between the electrodes is of such a nature that it is active on the polar particles constituting the medicament so as to carry them and cause penetration of same through the skin. Both these solutions however have many drawbacks, due above all to the action of the electric current passing between the electrodes. In fact, this current is sometimes particularly painful due to overheating of the skin and in addition, if not regulated in a correct manner, can cause burns on said skin.

It is further to be pointed out that the electrodes can cause passage of electric current also through the internal organs of the human body that are often sensitive to electric stresses. Therefore, passage of electric current is sometimes dangerous and inadvisable, particularly on patients sensitive to this type of treatment. It is also to be pointed out that transport of molecules through electric current only acts on the polar molecules of the medicament. Consequently, this treatment is limited to substances consisting of electrically charged molecules.

Finally, another drawback is given by the structural complexity and bulkiness of the above described device which involves use of the two electrodes disposed on opposite sides of the body.

Accordingly, it is an aim of the present invention to provide a medical apparatus for cutaneous administration of medicaments that is able to eliminate the above mentioned problems.

In particular, the present invention aims at providing an apparatus that can administer a medicament penetrating into the skin painlessly and without causing injury to the skin itself.

It is a further fundamental aim of the present invention to make available an apparatus capable of acting on medicaments consisting both of polar and nonpolar molecules.

It is also an additional aim of the invention to make available a machine enabling implementation of all the therapy steps in a simple and efficient manner.

The foregoing and still further aims that will become more apparent in the course of the following description are achieved by an apparatus for cutaneous administration of medicaments comprising the features recited in claim 1 and in the claims depending thereon.

Further features and advantages will be best understood from the detailed description of a preferred but non exclusive embodiment of an apparatus for cutaneous administration of medicaments according to the present invention.

This description will be set out hereinafter with reference to the accompanying drawings, given by way of non-limiting example, in which:
- Fig. 1 is a perspective view of a medical machine in accordance with the invention;
- Fig. 2a is a diagrammatic elevation view of an apparatus for cutaneous administration of medicaments made in accordance with the present invention;
- Fig. 2b is an exploded view of the apparatus seen in Fig. 2a;
- Fig. 3 is a diagrammatic elevation view of an apparatus for cutaneous administration of medicaments in accordance with a second embodiment of the invention.

With reference to the drawings, an apparatus for cutaneous administration of medicaments has been generally identified with reference numeral 1.

Apparatus 1 advantageously comprises a container 2 having a substantially tubular cylindrical extension and provided with a first aperture 2a and a second aperture 2b opposite to the first one. The container 2 is designed to hold a medicamentous solution 3 consisting of a preferably solid matrix, containing at least one first medicament. The medicament can be of different nature based on the curative action it must have on the human body; instead, the matrix is advantageously made up either of a gel or a hydrogel, or alternatively of water (distilled water, for example) or an aqueous solution that is made frozen and therefore brought to the solid state.

It is however apparent that any type of product enabling a medicament or an active ingredient to be made in solution and to be brought from a liquid phase to a substantially solid phase is adapted to the type of application hereinafter described (let us think of an oily solution or similar materials, for example).

As shown in the accompanying drawings, the container 2 at one of its ends close to the second aperture 2b has an end portion 4 adapted to be associated with a lid 6 through suitable coupling means 5.

In more detail, the lid 6 has a substantially cylindrical extension and is designed to shut the second aperture 2b of the container 2. Advantageously, as shown in the accompanying figures by way of example, the coupling means 5 consists of suitable screw threads formed on the inner surface of the lid 6 and the outer surface of the end portion 4. In this way the lid 6 can be removably associated with the end portion 4 to shut the second aperture 2b on filling of the container 2 with the solution 3 in the liquid state and can be removed from the end portion 4 in a use condition, when the medicamentous solution 3 has been brought to the solid state.

Apparatus 1 further comprises a piston 7 insertable into the first aperture 2a and having a substantially cylindrical conformation. In particular, piston 7 has an upper surface 7a adapted to abut against the medicamentous solution 3 held in the container 2, and a lower surface 7b opposite to the upper one 7a. Piston 7 that is slidable along the longitudinal axis of the container 2, further comprises a side wall 7c sealingly in engagement with the inner surface of the container 2.

In addition, a through hole 8 which is coaxial with said cylindrical container 2, is formed in piston 7.

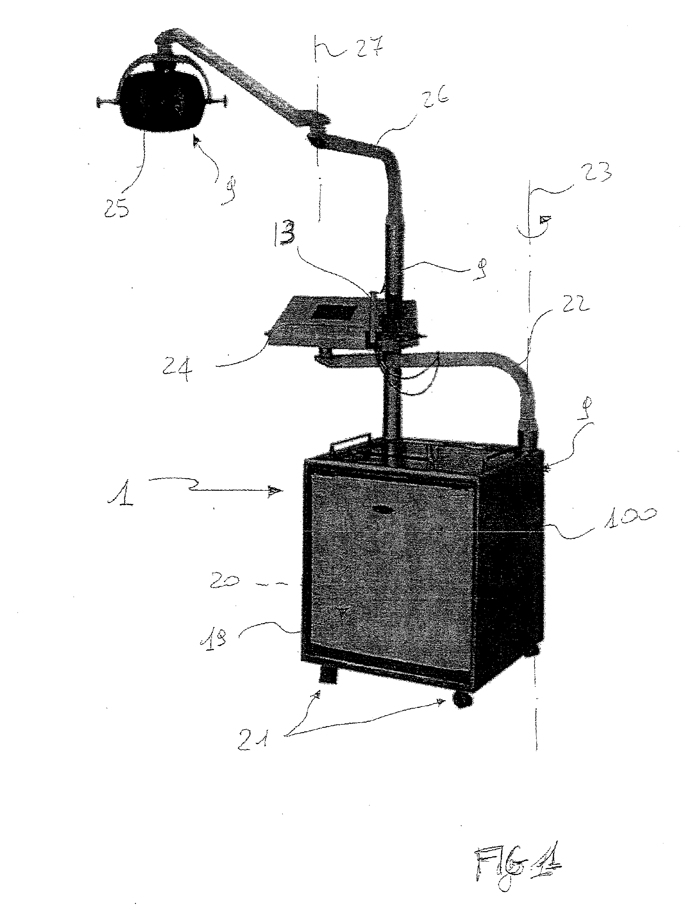

On thoroughly examining the machine from a structural point of view, the apparatus 1 shown in Fig. 1 consists of a supporting frame 100 provided with suitable actuating means 21 to enable displacement of the whole structure within an environment. In particular, for the purpose, this frame 100 will be equipped with a predetermined number of wheels.

As can be also viewed from Fig. 1, the medical apparatus comprises means 19 for freezing the medicamentous solution 3 or keeping it frozen at the solid state.

In particular, this means 19 comprises a holding space 20 shown in the figure and a refrigerating system adapted to generate the freezing temperature in the space 20 itself.

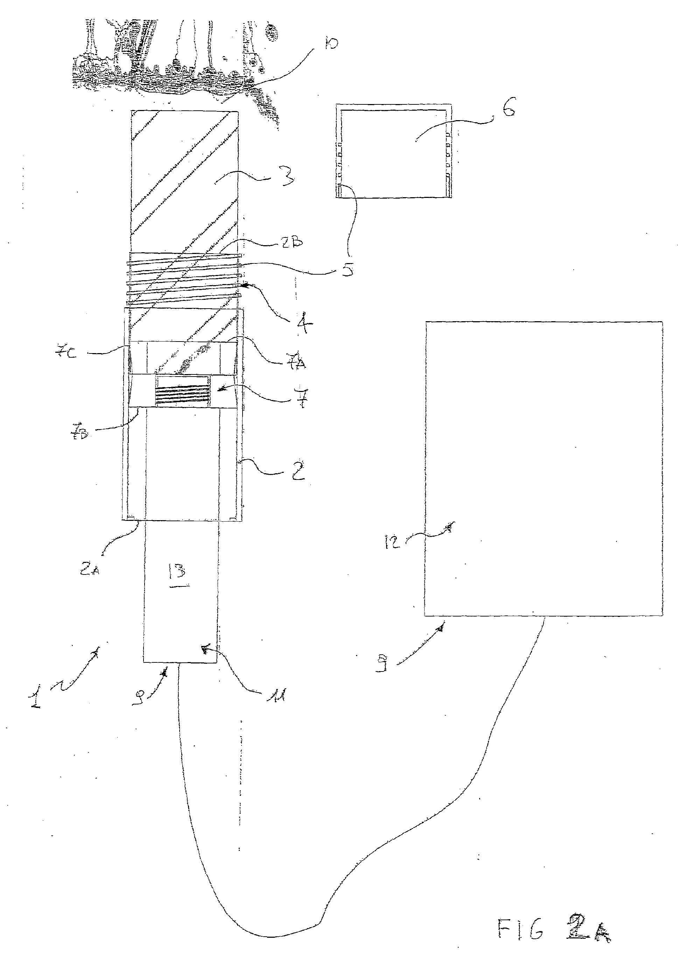

In particular, it will be possible to prepare the suitable medicament in the form of an aqueous solution or a gel and then put the container 2 shown in Figs. 2a to 3 in the holding space 20 until the solution has become fully frozen.

Still looking at Fig. 1 it is possible to also see the presence of a first movable arm 22 emerging from the supporting frame 100 and rotatably linked thereto around a pivot axis 23.

This first movable arm 22 is designed to support a command display 24 provided with a monitor and suitable for entering and/or monitoring the treatment being carried out and the control parameters of the apparatus itself.

The container 2 too and a handling device 13 to be described in the following are supported by the movable arm 22 close to the command display 24.

The mobile character of the first movable arm 22 combined with the actuating means 21 allows reaching of any region of the patient's body to be treated.

It is also possible to notice the presence of a second movable arm 26 also emerging from the upper surface of the supporting frame 100 and designed to support a suitable auxiliary radiation body 25 capable of sending electromagnetic waves (a laser light) at least towards the skin region to be treated.

In this respect, the second movable arm 26 is also shiftable in rotation about a further substantially vertical axis 27, while the auxiliary radiation body 25 can be oriented in different operating positions relative to the second movable arm 26 and the supporting frame 100.

Due to the above, an optimal orientation of the auxiliary body 25 relative to the skin region of the patient to be treated is once more obtained.

Apparatus 1 further comprises an energy emitter 9 active on the molecules of said medicament contained in the solution 3 to cause penetration of the medicament itself into a skin region 10 to be treated diagrammatically shown in Fig. 2a. It is to be pointed out that the energy emitter 9 is not an electric-energy emitter and therefore it does not emit any type of electric current.

In detail, the energy emitter 9 is active on both polar and non-polar molecules possibly present in the medicament and consists of an electromagnetic-wave generator 11 designed to emit luminous energy. In more detail, the electromagnetic-wave generator 11 that is of known type and therefore will not be further described, emits laser light and the emitted light has variable power and wavelength based on the type of treatment and the nature of the solution 3.
Furthermore, the light emission can be of the continuous or pulsed type, being directly supplied from the emitting source or conveyed through optical fibres to the medicamentous solution 3.

The electromagnetic-wave generator (incorporated in the frame 100 and therefore non visible in Fig. 1) comprises an energy source diagrammatically represented by block 12, and a handling device 13 preferably having a substantially cylindrical conformation. The handling device 13 has a first end 13a intended for emission of the laser light and a second end operatively associated with the energy source 12 by means of connecting elements of known type (diagrammatically embodied in the accompanying figures by an optical cable).

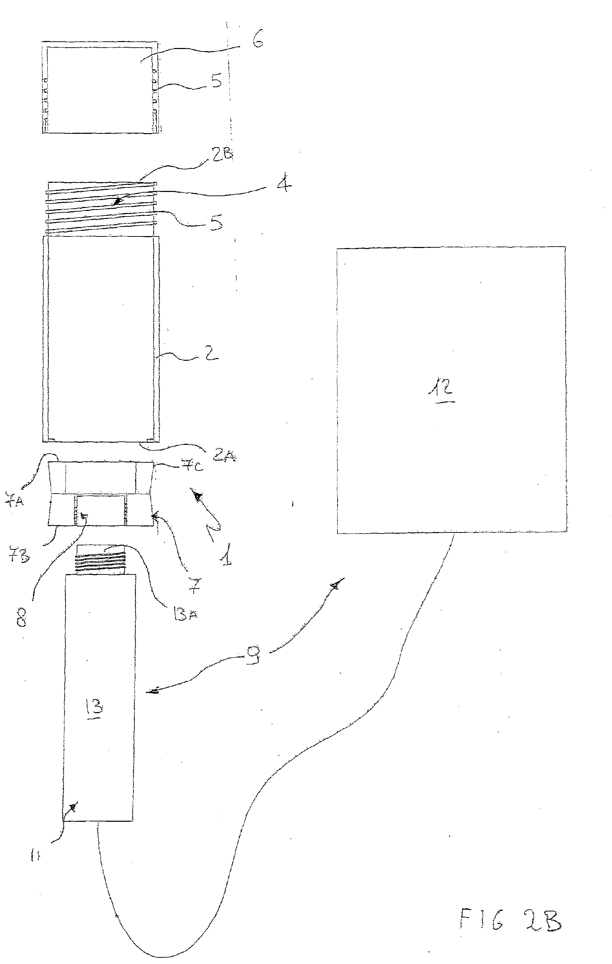

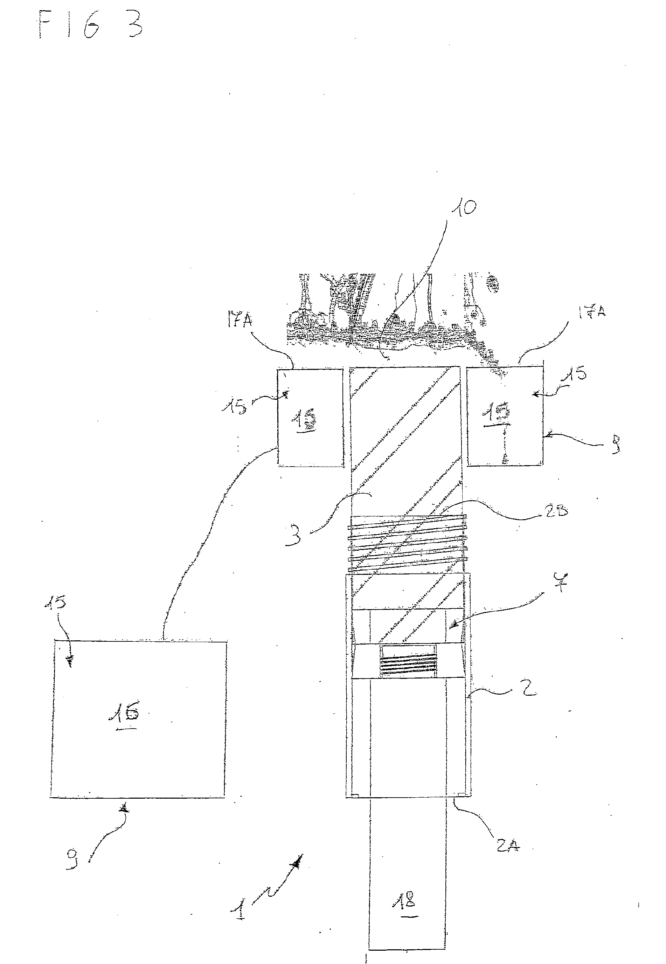

The first end 13a of the handling device 13 is operatively in engagement with the lower surface 7b of piston 7. In particular, as shown in Fig. 2a, the first end 13a is associated with the through hole 8 of piston 7 through suitable connecting means of known type, such as a threaded connection. Under this situation, the handling device 13 and related piston 7 are slidable in the container 2 along the longitudinal axis of the container 2 itself, to move the solid medicamentous solution 7 out of the second aperture 2b during operation of the apparatus (Figs. 2a and 3).

It is to be pointed out that, as shown in Fig. 2a, the laser light emitted from the first end 13a of the handling device 13 is projected along a direction parallel to the longitudinal extension of the container 2 and is free to pass through the through hole 8 to interact with the solid medicamentous solution 3.

In accordance with a second embodiment shown in Fig. 3, the energy emitter 9 is made up of a sound energy generator 15. More particularly, the sound energy generator 15 is an ultrasonic wave generator of a well known type and therefore not further described in detail.

The ultrasonic wave generator 15 comprises a source of sound energy diagrammatically represented by block 16, and an annular body 17 in engagement with said source 16 through suitable known means.

The annular body 17 is disposed close to the second aperture 2b and is coaxially external to the solid medicamentous solution 3. In addition, the annular body 17 has an active surface 17a designed to emit ultrasonic waves, that can be brought into abutment against the skin region 10 to be treated.

In this embodiment, a handling device 18 insertable in the first aperture 2a of container 2 is also provided; it has an upper surface susceptible of abutment against the solid solution 3 to cause movement of the solution 3 and make it come out of the second aperture 2b.

Finally, the energy emitter 9 can also consist both of the ultrasonic wave generator 15 and of the electromagnetic wave generator 11 of the above described type. Under this situation, the molecules present in the medicament contained in the solution 3 are submitted both to the light radiation of the laser generator 11 and to the sound radiation of the ultrasonic wave generator 15.

Therefore it is to be noted that the energy emitter 9 also comprises the auxiliary radiation body 25 described above.

In fact, the laser light can be conveyed by the same electromagnetic wave generator 11 (or possibly by a second laser light generator) to this body too in order to improve the treatment, as better explained in the following.

After the above description from a structural point of view, operation of the apparatus in accordance with the invention is as follows.

The medicamentous solution 3 at the liquid state is introduced into the container 2 that is closed with the suitable lid 6. The container 2 is then cooled in the holding space 20 until bringing the solution 3 to the solid state, which solution preferably containing a water matrix or an aqueous solution becomes frozen.

An improvement in the passage properties of the medicament is also highlighted if the solution 3 is brought to temperatures included between -15 and -22 degrees and preferably to -18°C.

At this point, the lid 6 is removed and by the action of the handling device 13 on piston 7, the solution 3 is urged out of the second aperture abutting against the skin region 10 to be treated. In this manner the solution 3 being in contact with the skin heats up and releases a liquid film on the skin itself.

In accordance with the first embodiment, the laser light emitted from the first end 13a of the handling device 13 passes through the solution 3 and strikes on the skin region 10. Under this situation, the aqueous matrix does not suffer modifications because the photons constituting the laser light will only act on the molecule crystals constituting the medicament. These molecules are therefore pushed and introduced through the epidermis into the skin region 10 to be treated. Based on the type of treatment and the medicament molecules present in the cryogenic handling device, the emitted laser light will be the most appropriate to act on the molecules themselves.

For instance, according to the Applicant's findings it is particularly advantageous to use an escina-based medicamentous solution which is treated with a laser light of a wavelength in the range of 600 to 650 mm and preferably of 635 mm.

Likewise, in accordance with the second embodiment, the medicament molecules are submitted to the action of the sound waves emitted by the annular body 17.

In detail, the film of liquid solution 3 is disposed on the skin region to be treated 10 under the annular body and is consequently radiated by the ultrasonic waves pushing the medicament molecules into the skin pores.

Once the medicament molecules have penetrated the skin pores, a further scanning carried out on the region to be treated by use of the auxiliary radiation body 25 appears to be very advantageous. In particular, a further laser light is caused to strike on the region being treated (this time without use of the handling device 13).

The last mentioned action enables better distribution of the medicament penetrated into the human skin following application by means of the handling device 13 and therefore increases the curative efficiency of the treatment.

The present invention achieves important advantages.

First of all, the apparatus 1 does not cause burns on the skin. This advantage is due both to the cooling action of the medicamentous solution 3 that is frozen and to the effect of the laser beam that does not damage the skin.

A further advantage is given by the great number of applications of the sound and electromagnetic energy on any medicament. In fact, both the laser light and the ultrasonic waves act on the non polar and polar molecules of the medicament itself. Consequently, there is a wide possibility of use for each type of medicament that is utilised for each respective pathology. In addition, a further advantage is given by the structural simplicity of the present apparatus in which, in contrast to the electrophoresis therapy in which two electrodes are employed, use of a simple light and/or ultrasonic wave generator of known type is required.

## Claims

1. A medical apparatus for cutaneous administration of medicaments comprising:
- a supporting frame (100);
- an energy emitter (9) in engagement with the frame (100) and active on the molecules of at least one medicament to cause penetration of same into a skin region to be treated (10); and
- a medicamentous solution (3) adapted to be positioned between the energy emitter (9) and the skin region to be treated (10), said solution (3) comprising a matrix containing said medicament,
**characterised in that** said energy emitter (9) is not an emitter of electric energy.

2. An apparatus as claimed in claim 1, **characterised in that** said energy emitter (9) is active both on polar molecules and non-polar molecules present in the medicament.

3. An apparatus as claimed in claim 2, **characterised in that** said energy emitter (9) is a generator of electromagnetic waves (11) emitting luminous energy, preferably laser light.

4. An apparatus as claimed in claim 1, **characterised in that** it further comprises a container (2) to hold the medicamentous solution (3), said solution (3) being at the solid state and slidable along a longitudinal axis of the container (2).

5. An apparatus as claimed in claim 1, **characterised in that** it comprises means (19) for freezing the medicamentous solution (3) at the solid state or maintain it in a frozen condition, which means (19) is linked to said supporting frame (100) and preferably comprises a holding space (20) and a refrigerating system to generate a freezing temperature in the holding space (20).

6. An apparatus as claimed in claim 1, **characterised in that** it further comprises actuating means (21) in engagement with the frame (100) to enable displacement of the apparatus, said actuating means comprising wheels, for example.

7. An apparatus as claimed in claim 1, **characterised in that** it comprises a first movable arm (22) carried by the supporting frame (100) and designed to support said energy emitter (9).

8. An apparatus as claimed in claim 7, **characterised in that** the first arm (22) is movable in rotation around a preferably vertical axis (23).

9. An apparatus as claimed in claim 8, **characterised in that** it comprises a command display (24) for entering and/or monitoring the control parameters.

10. An apparatus as claimed in claim 1, **characterised in that** said energy emitter (9) further comprises an auxiliary radiation body (25) designed to send electromagnetic waves at least towards the skin region to be treated.

11. An apparatus as claimed in claim 10, **characterised in that** it comprises a second movable arm (26) carried by the supporting frame (100) and designed to support said auxiliary radiation body (25).

12. An apparatus as claimed in claim 11, **characterised in that** the second arm (26) is movable in rotation around a preferably vertical axis (27), the auxiliary radiation body (25) being susceptible of being oriented in different operating positions relative to the second arm (26) and the supporting frame (100).

13. An apparatus as claimed in claims 3 and 4,
**characterised in that** it further comprises a piston (7) slidable in said container (2) and preferably insertable in a first aperture (2a) of the container (2), said piston (7) having an upper surface (7a) adapted to abut against said solution (3) and a lower surface (7b) to be operatively engaged with said electromagnetic wave generator (11).

14. An apparatus as claimed in claim 13, **characterised in that** said electromagnetic wave generator (11) comprises a handling device (13) having a first end (13a) designed to emit the energy and adapted to be associated with the inside of a through hole (8) formed in said piston (7), said handling device (13) and piston (7) being slidable in said container (2) to shift the solid medicamentous solution (3) out of a second aperture (2b) of the container (2) opposite to the first aperture (2a).

15. An apparatus as claimed in claim 4, **characterised in that** it further comprises a cylindrical lid (6) to be associated with the second aperture (2b) of the container (2) to shut said second aperture (2b) in a condition of the container (2) in which it is filled with the solution (3) at the liquid state and to be removable from the second aperture (2b) in a condition of use in which the solution (3) is converted to the solid state.
